# EUROPEAN PATENT APPLICATION

(11) **EP 4 397 742 A1**
(43) Date of publication of application: **10.07.2024**
(21) Application number: 23217905.1
(22) Date of filing: 19.12.2023
(51) Int. Cl.: C12M 1/00, C12M 1/36

(54) **METHOD FOR GROWING MICROALGAE**

(30) Priority: 21.12.2022 IT 202200026289
(71) Applicant: NORD ENERGIA S.p.A. in liquidazione, 20123 Milano (IT); Semmel S.r.l., 20154 Milano (IT)
(72) Inventor: Merli, Pietro Giacomo Maria, 20137 Milano (IT); Quadrio, Maurizio, 23036 Teglio (SO) (IT); Chiarini, Alessandro, 00189 Roma (IT); Dini Ciacci, Gabriele, 20154 Milano (IT)
(74) Representative: Rastelli, Franco

(57) **Abstract**

A method for growing biomass particles, in particular microalgae, in a culture medium and in the presence of one or more light and nutrient sources, which provides for space-time control of said biomass particles with respect to said light and nutrient sources in order to optimize and maximize the growth rate of the biomass. In relation to prior art technologies, the method of the invention allows control of the process parameters, respectively "space", meant as position of the particles relative to the light and nutrient sources, and "time", i.e., the time for which the particles remain exposed to light and nutrients, so as to promote the biochemical reactions necessary for the growth of these biomass particles.

## Description

### BACKGROUND OF THE INVENTION

The present invention concerns a method for growing biomass in the form of particles, in particular microalgae.

Microalgae are plant organisms, used in the energy sector as base for the production of biofuels, in the field of nutraceuticals, for example food supplements, in the pharmaceutical and cosmetics sectors.

In particular, within the scope of microalgae growth, it is known to disperse the biomass in water, by supplying light, CO₂ and nutrients useful for growth.

In the prior art, the supply of light does not consider the absorption spectrum of the single algal species and in particular the emission spectrum is not varied during growth. Rarely, some systems vary the intensity of the light source, mainly by switching the source on or off completely. Other systems vary the light intensity over time in a predetermined manner, without dynamic control and without considering the system parameters [https://doi.org/10.1007/s10529-018-2595-3]. Yet other systems carry out static filtering, only of the intensity or blocking part of the spectrum, of the light source [US2014011263A1].

With regard to dissolved CO₂ and nutrients, the prior art does not apply any dynamic control to ensure that these parameters remain within a given desired range. Almost all the systems input these substances constantly, either at the beginning or at the end of the process. More rarely, other systems provide for a predetermined variation of the input amount.

Therefore, it is fundamental for efficient volumetric accretion of the microalgae, i.e., an accretion extended to the whole of the volume of water and biomass treated, to be able to supply light and nutrients not only in the correct proportions, but also in the right space-time frequency distribution. To be able to supply what is required in the right space-time frequency distribution, correct tracking not only of the dynamics of the fluid, exemplified by the streamlines, but also individual tracking of the individual algal particles, exemplified by the pathlines, is necessary.

In fact, the microalgae accretion process involves, with regard to space, the possibility of allowing the particles of the algal mass to be in the best position to receive light and nutrients and, with regard to time, that exposure to light is compatible with the natural biological times of the specific organism. Best position is meant as the capability to identify and track the spatial coordinates of the individual biomass particles. Exposure time is meant as the capability to control intensity, exposure frequency and wavelength of the light sources affecting the individual particles.

In fact, with regard to the biological and chemical growth times of the algae, these must reproduce an alternation of light and dark, according to the natural growth cycle of these organisms. In particular, illumination of microalgae should take into account the different wavelengths, necessary for the activation reaction of photosynthesis precursors, in particular carotenoids and chlorophyll. In the prior art, the biological times are set for the whole culture medium and are therefore the same for all the individual particles that are exposed to the different conditions in a substantially random way [https://doi.org/10.1007/s10811-020-02310-1].

Currently, the techniques used are limited to maintaining under strirring the biomass dispersed in water, so as to bring the microalgae as much as possible into contact with the light source and with the nutrients. However, the operation is carried out through simple stirring, in a disorderly way, i.e., without any space-time control on the location of the microalgae with respect to the environment, on the exposure times to the light for different wavelengths and to nutrients. Consequently, in the prior art the chaotic flow controls and regulates the exposure times of the particles to the light within the culture medium. This is also the case for the spectrum to which the particles are exposed, which is a consequence of their spatial position over time due to the effect of spectral absorption given by the other particles interposed with the light source [https://doi.org/10.1007/s10529-018-2595-3].

Therefore, with traditional techniques, growth of the mass of microalgae in volumetric terms is implemented in an uncontrolled and completely random manner. For this reason, accretion of the algal mass is shifted toward the irradiated surface, while it is lower or more limited within the volume of the biomass in a position far from the light source and from the nutrients. Completely random behaviour is meant as lacking a form of individual tracking of the particles, which are considered dispersed and simply transported in turbulent motion.

The publication by Gao Xi et al. "Simulation of algal photobioreactors: recent developments and challenges" in BIOTECHNOLOGY LETTERS, KLUWER ACADEMIC PUBLISHERS, DORDRECHT, vol. 40, no. 9, 26 July 2018, pp. 1311-1327 [https://doi.org/10.1007/s10529-018-2595-3], concerns a method for growing microalgae for treating wastewaters and for reducing CO₂.

The publication by Chiarini Alessandro et al. "The light/dark cycle of microalgae in a thin-layer photobioreactor" in JOURNAL OF APPLIED PHYCOLOGY, vol. 33, no. 1, 19 November 2020, pp. 183-195 [https://doi.org/10.1007/s10811-020-02310-1], discloses a study on the transport of algae in a photobioreactor.

The publication by Papácek Stepán et al. "Towards Integration of CFD and Photosynthetic Reaction Kinetics in Modeling of Microalgae Culture Systems", 1 April 2017, SAT 2015 18TH INTERNATIONAL CONFERENCE, AUSTIN, TX, USA, SEPTEMBER 24-27, 2015; [LECTURE NOTES IN COMPUTER SCIENCE; LECT. NOTES COMPUTER], SPRINGER, BERLIN, HEIDELBERG, pp. 679-690, discloses a multidisciplinary model for linking the growth of microalgae to the optical and hydrodynamic conditions of the environment.

### SUMMARY OF THE INVENTION

The main object of the present invention is to provide a method suitable for improving volumetric accretion of the biomass particles, in particular of microalgae in a fluid, for example in water, respecting the parameters of the biological growth cycle of the microalgae, in terms of exposure to light and to nutrients, with particular regard to the different algal species. Respecting the parameters of the biological cycle is meant as the capability to provide the biomass with a given combination of the required parameters optimal to achieve different goals. This includes adaptation of the light emission spectrum to the absorption spectrum of the species or of the combination of species of biomass present in the volume.

This and other objects are achieved with the method of claim 1. Preferred embodiments of the invention are set forth in the remaining claims.

In relation to known technologies, the method of the invention allows the use of the process parameters, respectively "space", meant as position of the alga relative to the light and nutrient sources, and "time", i.e., the time for which the alga remains exposed to light and nutrients, so as to promote the biochemical reactions necessary for the growth of these biomass particles. The light sources are a set of multiple and different emitters of light radiation with individually controllable intensity, wavelength and frequency in relation to "space" and to "time" and therefore capable of producing a state of illumination that is the combination of the superposition of these emitters. The nutrients are the set of substances dissolved in the liquid, the concentration of which can be controlled individually in order to obtain a culture medium with a given combination of parameters.

The invention in particular offers the advantage of dynamically controlling the aforesaid parameters, i.e.:
- the position in space, given by the pathlines, of the microalga relative to the light and nutrient sources, through the formation of vortices in the fluid in question, such as the culture medium, managed by number and by speed;
- the temporal conditions of exposure of the microalgae to light, distinguishing by wavelength, intensity and temporal frequency of exposure.

These parameters are controlled, as stated above, through variation of the velocity and the number of vortices, of the light meant as combination of the light emissions of multiple and different emitters, through prediction of the position of the particles by means of fluid dynamic methods, through measurement of the parameters that allows identification of the status of the system according to calculation models and measurement of the dissolved substances. It is also possible to alter the state of the biomass by influencing the amount of carbohydrates, the amount of fats and the concentration of other substances within the biomass particles depending on the application required.

### BRIED DESCRIPTION OF THE DRAWINGS

These and other objects, advantages and features will be apparent from the following description of a preferred embodiment of the method of the invention illustrated, by way of non-limiting example, in the figures of the accompanying drawings.

Wherein:
- Fig. 1 illustrates an embodiment of an open pond for growing microalgae;
- Fig. 2 illustrates, in schematic form, the mechanisms of space-time control of the method of the invention;
- Fig. 3 illustrates the detail of a vortex of the diagram in Fig. 2; and
- Fig. 4 represents the block diagram of the method showing the mathematical models used for resolution of the flow field of the culture medium, the tracking of the microalgae within the culture medium, the radiative model, the microalgae growth model, the calculation of the dose of light acquired by the microalgae, the identification of the status of the system according to these models and the interaction between these models.

### DETAILED DESCRIPTION OF THE PREFERRED EMBODIMENTS

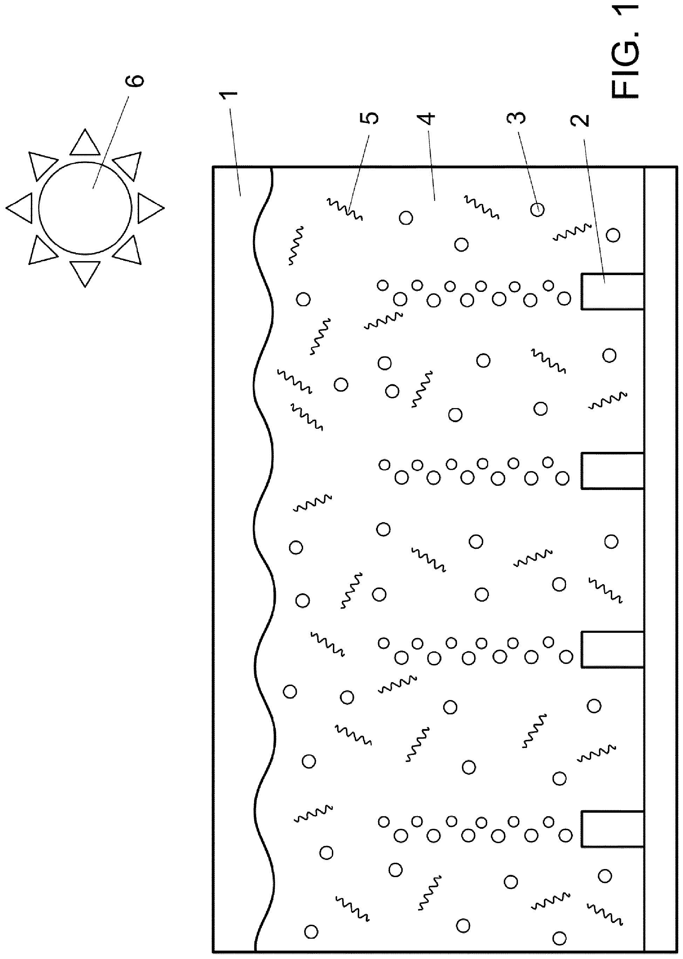

The pond used for the growth of biomass particles according to the prior art, as illustrated in Fig. 1, is indicated as a whole with the reference number 1 and has, on the bottom, dispensers 2 of nutrients, both liquid and gaseous, in the example illustrated in the form of beads 3.

A culture medium 4, substantially consisting of water, in which the biomass particles, in the example illustrated microalgae 5, are dispersed, is placed inside the pond 1. In this embodiment, the illumination used is the sunlight 6.

From the above it is understood that the supply of light and nutrients to the microalgae 5 is random and limited, without any space-time control. In fact, the position of the microalgae relative to the light and to the nutrients dissolved in the fluid, and their exposure time to these, are not subjected to any control, but depend solely on the entirely random position of the microalgae relative to these light and nutrient sources. The position is random for two reasons: the first is that the biomass particles are immersed in a turbulent flow; the second is that no identification is carried out on the status of the system and there is no control based on the parameters measured. In the prior art, the particles are considered dissolved and modelled as following the streamlines and evaluated in an aggregated and statistical manner, without considering their individual history.

Consequently, not only is the growth of the microalgae not controlled in its evolution, but it actually stops when the biomass on the surface blocks access of the light to the lower layers of the biomass, preventing the natural biological growth cycle from taking place in these layers. At the same time, the free surface of the biomass is inhibited due to overexposure to sunlight causing the production of inhibitory substances by the microalga. These substances are then dissolved in the whole of the culture medium, thereby slowing down and eventually preventing the natural algal growth process in the whole of the volume. These drawbacks that hinder growth can be considered a consequence of the substantial asymmetry, inhomogeneity and lack of control of the space-time distribution of the microalga, of the light and of nutrients within the biomass.

To overcome these drawbacks, it is known to apply simple mixing of the culture medium; however, this creates a state of disorder that does not allow effective control of the growth conditions of the biomass particles. On the contrary, according to the teachings of the prior art, simple mixing also includes mixing that uses a turbulent flow, which by fluid dynamic definition is substantially chaotic; this simple mixing can lead to an increase in productivity, which however is limited, being substantially correlated to a mixing of statistical type without active control and without taking into account the history of the particles and the actual status of the system [https://doi.org/10.1007/s10811-020-02310-1].

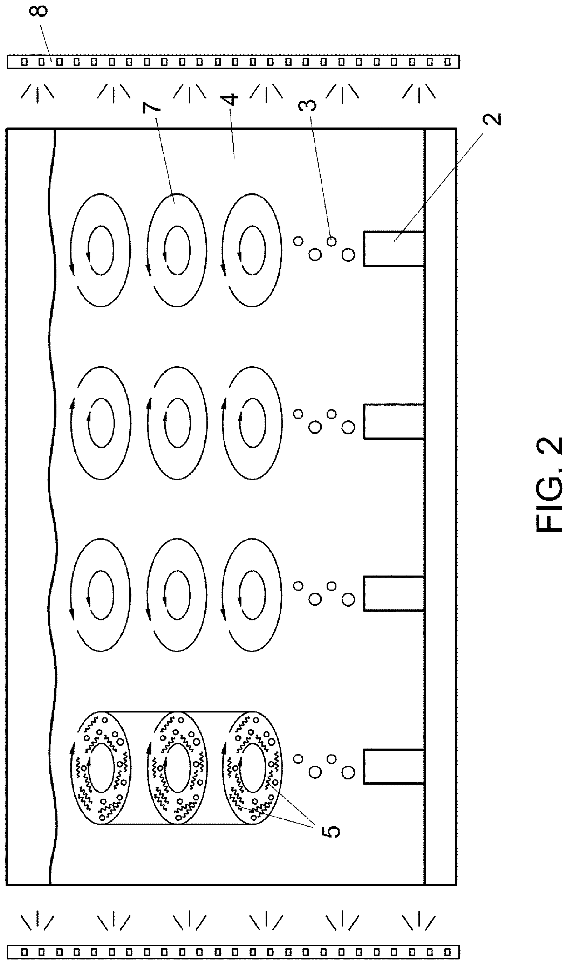

The aforesaid drawbacks of the prior art are overcome with the method of the invention, as schematized in Fig. 2, according to which vortices 7 that entrain the microalgae according to a numerically predictable trajectory are created within the culture medium 4.

Advantageously, according to the invention said vortices 7 are vortices in laminar flow, which can be controlled in terms of position relative to the nutrients 3 and to the light sources 8, and in terms of time, i.e., of exposure of the microalgae with respect both to light and to nutrients. The vortices, generated with different techniques, are laminar of three-dimensional nature, for example as defined in the literature [https://doi.org/10.1007/s003480050071], characterized by the possibility of predicting the precise position of the individual particles.

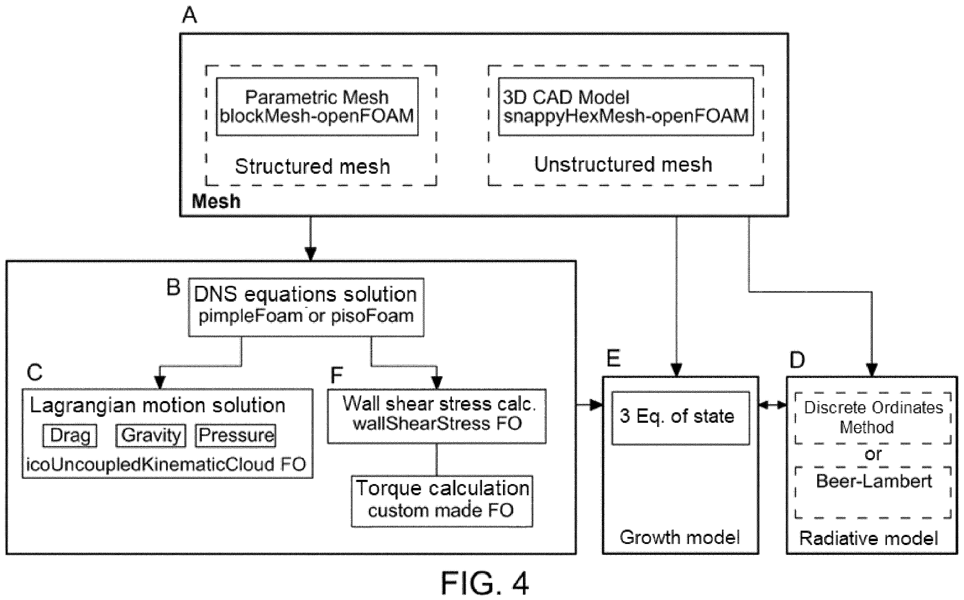

At the same time, the vortices 7 produce the necessary mixing of the biomass, fractionating it so as to make it wholly available towards the light and the nutrients, giving the system the necessary homogeneity. This fractionation takes due account of the shear stress to which the algae are subjected, as indicated in Fig. 4 with F.

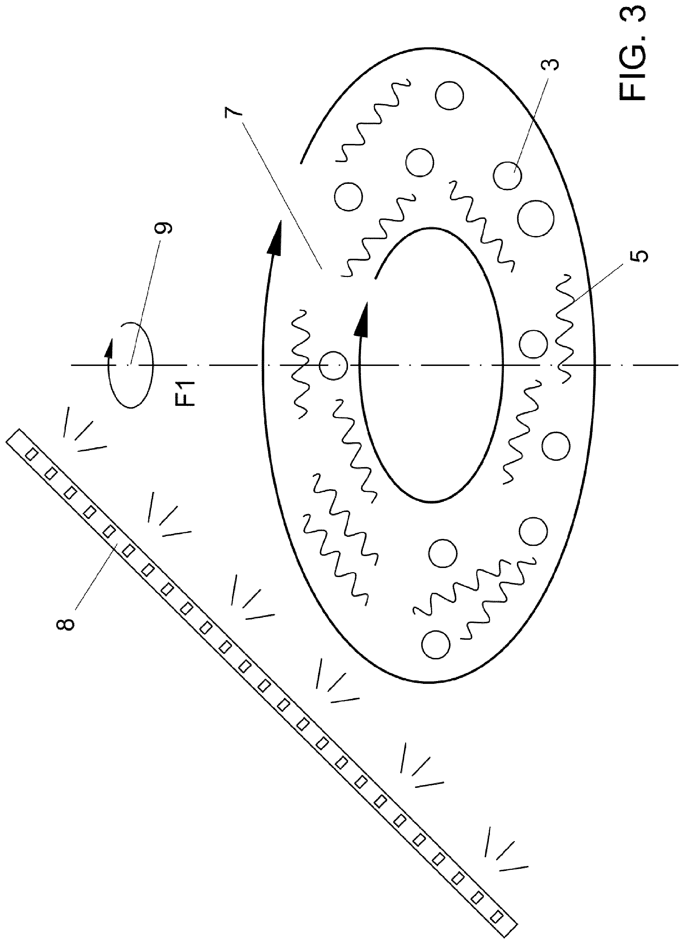

As better illustrated in Fig. 3, the vortex 7 in laminar flow has a rotation F1 about an axis 9, either curved or straight.

The different calculation steps of the parameters used in the method of the invention are described below highlighting, by way of non-limiting example, the type of calculation models that can be used. The calculation models are the set of instruments capable of returning the values of the parameters required to execute the control method which is part of the innovation disclosed with the present invention. These are currently:

### I) Modelling of the geometry of the vortex forming device

In the diagram of Fig. 4, box A represents the modelling of the geometry of the vortex forming device 7, within which the flow field is calculated. For the purposes of this modelling, a mesh, i.e., a numerical discretization, suitable to describe the geometry of the domain of the flow field of the culture medium 4, is produced. This can be made with the structured or unstructured mesh generators available in the software OpenFOAM, as better described below.

### II) Calculation of the flow field of the of vortex formation volume of the fluid

A vortex is a fluid region in which the flow rotates about a rectilinear or curved axis. Vortices are a relatively common phenomenon, characterized mainly by the presence of turbulent flows. In fact, a turbulent flow can also be viewed as the superposition of a myriad of vortices of different sizes and of different vortex scales. In particular, vortices are characterized by defining in the flow field the velocity distribution, the vorticity (through the rotor) and the circulation concept. Vortices can be both two-dimensional and three-dimensional, stationary and non-stationary.

A particular type of vortex is the stationary vortex which can occur in a laminar stream. This vortex is an idealization of a real phenomenon, in which a vortex does not substantially vary its position in space and in which the fluid particles of which it is composed flow in an orderly fashion on top of one another with substantial absence or limitation of the chaotic phenomena that correspond to turbulent flows. Therefore, this type of configuration assumes specific statistical characteristics with regard to the motion of the particles. In fact, it must be remembered that the difference between a turbulent flow and a laminar flow is linked to the scale of the vortices, which range from the Kolmogorov scales to the integral scales.

The absence of the smallest Kolmogorov scales leads to low energy dissipation and therefore characterizes a laminar vortex as a structure that has little energy dissipation. However, the same structure is still able to transport momentum and energy, which is a feature of larger vortices. Both these features are useful to the invention.

Vortex calculation can be performed using the software OpenFOAM available on the website https://openfoam.org, and in the literature in the article available at the address [http://dx.doi.org/10.1063/1.168744] (Fig. 4, box B), using a DNS (Direct Numerical Simulation) model, i.e., a computational fluid dynamic simulation model in which the Navier-Stokes equations are solved completely and without the use of an approximate turbulence model. Given the type of flow of interest and from the results of the DNS investigations, it is also possible to perform simulations with simplified models already known in the literature, such as LES (Large Eddy Simulation, https://en.wikipedia.org/wiki/Large_eddy_simulation) [https://doi.org/10.1063/1.868436], [https://doi.org/10.1007/b137536], obtaining a sufficiently accurate prediction of the flow fields and of their characteristics, sufficient for the use in this invention. The model utilized is the model for incompressible fluids, both single-phase and two-phase, to take into account the possible and unnecessary presence of air bubbles.

### III) Tracking of the biomass particles

According to the invention, the microalgae 5 that are immersed in the fluid that fills the pond 1 can be tracked with specific mathematical methods that calculate their dynamics, i.e., the position as a function of time within the flow field. In particular, for the invention the algal particles within a flow in the presence of vortices 7 as described above (Fig. 4, box C) were tracked.

The mathematical methods used to track the biomass particles formed by the microalgae are based on what is provided in the software OpenFOAM and modifying the Lagrangian Particle Tracking model (LPT method) to consider the characteristics of the different biomass particles dependent on species [https://doi.org/10.1146/annurev-fluid-031822-041721].

Part of the innovation of this method is to take into account the characteristics of the individual particles such as mass, shape, absorption profile of light radiation, different photosynthesis cycles, different composition of the biomass as a whole, growth status and photosynthetic status and to apply the growth and status models individually.

The model utilized in the present invention considers the particles with their mass and density, forces due to fluid dynamic resistance, buoyancy forces, volume forces and pressure forces. The chemical and electromagnetic forces and impacts between particles have been ignored and fluid-particle coupling is of one-way type. More complex methods, which improve the prediction of the position of the particles and the accuracy of the data collected, but lengthen calculation times, are also suitable for the invention.

From individual tracking of the particles the position in space as a function of the time within the flow field is obtained, also in the presence of vortices. Studies carried out showed that it is not necessary to track each individual biomass particle, but it is sufficient to track a large number to obtain reliable data. Data are considered reliable if a given group of particles behaves in a similar way for a time greater than the time after which recalculation of the flow field and of the mathematical model is performed as a result of the changed status of the system.

### IV) Calculation of the radiative model

After tracking the position of the microalgae within the culture medium, the light intensity present in each point thereof must be identified. Among the possible calculation models, by way of non-limiting example, the Discrete Ordinates Method, (DOM - Fig. 4, box D) and an analytical model based on the Beer-Lambert law were used.

Illumination of the fluid can be calculated through a radiative model of the light. The model utilized is the one present in the software OpenFOAM and is the Discrete Ordinates Method. This returns the light intensity and light spectrum in each point of the fluid.

Calculation was also tackled with a simplified analytical model of exponential type based on a Modified Extended version of the Beer-Lambert law. The modifications take into account the different absorption of the materials [https://doi.org/10.3390/app9061127], the corrections required for the law to give reliable results with high powers [https://doi.org/10.1364/AO.47.005354] and the correction due to the variable concentration of the biomass particles [https://doi.org/10.1142/S1793545814500266].

Although the results obtained with the two methods differ, giving different control parameters, the choice of one or the other does not interfere with the teaching of the present invention. The difference indicates that it is possible to obtain the control parameters with different models, but potentially creating sub-optimal controls typical of the prior art without control or based on a preset non-dynamic control that is not dependent on the status of the system.

### V) Calculation of the growth model of the biomass particles

The algal growth model is based on a system of three differential equations representing the status of the system meant as amount of biomass capable of reproducing, amount of inhibited biomass, i.e., not capable of utilizing the incident radiation, and amount of resting biomass. The model is experimentally validated by means of three multiplicative constants of the three states. The parameters calculated by the preceding models being known, this system allows the growth of the alga to be predicted. The growth model is applied to the individual particles and not to the whole system.

An estimate of O₂ production and of CO₂ consumption is also obtained as output through knowledge of the flow field and the algal growth model.

Knowledge of the flow field, the position of the biomass particles and the light intensity in each point of the flow field allows, through numerical integration, calculation of the dose of light to which each particle is exposed. The dose of light contains and tracks for each particle the following information: intensity, amount, wavelength and frequency of the light flow.

### VI) Calculation of the shear stress on the cell structure of biomass particles of different type and at different growth stages

Box F in Fig. 4 indicates the step of calculating the shear stress on the cell structure of the microalgae according to the traditional methodology [https://doi.org/10.1016/j.biotechadv.2018.03.001]. In particular, calculation is performed in the maximum gradient area of the flow velocity of the algal culture medium (e.g. https://www.openfoam.com/documentation/guides/latest/doc/guide-fos-field-wallShearStress.html). Shear stress is also calculated on the individual particles.

According to the invention, the use of all the quantities calculated above has allowed the introduction of a new space-time closed loop control system of the biomass with respect to the light and nutrient sources. Contrary to what is known, this control is capable of generating and maintaining optimal growth conditions for the particular biomass and of adapting them during growth. Moreover, it is possible to produce at different rates, for example, of maximum energy or volumetric efficiency based on the fluctuations in market demand.

In particular, these control parameters are the space-time distribution of the biomass, the space-time distribution of the nutrients, the space-time distribution of electromagnetic radiation, the concentration of CO₂ and O₂ dissolved in the vortex stream, the light intensity of the LEDs illuminating the biomass, the wavelength of the light, the switch on frequency of the light and its "duty-cycle", i.e., the ratio between the time during which the lighting is active and the time during which it is deactivated in a given period, the rotation speed of the vortices and their number, the presence or absence of vortices, the transition of the stream from laminar to turbulent, the quantity of nutrients introduced into the culture medium, the pH of the culture medium, the temperature of the culture medium. Maintenance of the correct dilution ratio of the fluid (generally water) in the culture medium is also controlled.

Traditional methodologies for forming vortices in laminar flow within a fluid can be used to implement the method of the invention. For example, cavity flow is suitable to form the vortices according to the invention (e.g. https://www.openfoam.com/documentation/tutorial-guide/2-incompressible-flow/2.1-lid-driven-cavity-flow) [https://doi.org/10.1016/0167-6105(90)90219-3], [https://catalog.princeton.edu/catalog/998208393506421],
[https://doi.org/10.1017/S0022112071000181].

By way of example, being able to calculate the trajectory of the particle in the fluid dynamic field used with respect to references consisting of the geometric coordinates and to the initial time, and the space-time distribution of the light intensity being known, it is possible to predict the position and the dose of light absorbed at each interval of time. In a completely simplified manner and for the sole purpose of explaining the more complex calculation methodologies used in the present invention, a particle P that moves on a trajectory of length L with velocity V is considered, an interval of time T being taken and the light intensity I for the same particle being known, it is possible to calculate the dose of light as D = T * I. The time employed T = L / V being known, by substitution D = L / V * I is obtained.

By way of example, the space-time control system that integrates and connects the following sub-systems of which it is formed is part of the invention:
1. **Space-time control theory.** Defines the desired specific growth rate of biomass and biomass species type or mixture of types.
2. **Definition of design operative point.** Determines targeted biomass growth rate and biomass species type or mixture.
3. **Biomass model.** Calculates the required lighting dose in terms of intensity and wavelength based on biomass targeted growth rate. Then selects the absorption spectrum according to the biomass species type and lastly reports the allowable shear stress range for the biomass species.
4. **Chemical model.** According to the composition of the solution, required medium types and current concentrations the model calculates the system pH and indicates inflow parameters needed to keep the reactor in the target state.
5. **Flow field solver.** Calculates incompressible flow. Determines vortex formation locations and their procession velocity, including axial and transversal velocity. Calculates shear stress from velocity gradients and compares it with the critical range defined by the biomass model.
6. **Radiation solver.** Receives radiation requirement in terms of dose of light dose, spectrum pattern, frequency and intensity data from the biomass model, vortex location and particles location from flow field solver. With these parameters outputs the light source model in terms of light intensity, wavelength, frequency and combination pattern of each light source.
7. **Identification system.** Identifies the status of the reactor from input given by reading of the system sensors. Maps the reactor status to the models and sets all the outputs of the system. In case of initialization also acts as a system initializer taking output from default settings.
8. **Closed-Loop feedback system.** Collects the data from the model and from the status of the system and then calculates the adjustments to apply to the system.
9. **Parameter limiting system.** The system estimates the error of identification by comparing real-time sensor data with calculated space-time model data. This system prevents divergence by limiting control parameter variation in case a large change would bring the reactor out of a stable status.
10. **Biomass density estimator.** Calculates biomass density from sensor readings. The input is an array of sensors: optical density with different wavelength and an optical particle counting system. The output is the estimated density of the biomass in the reactor. If the biomass is composed of multiple algae species, the system also estimates the ratio of the two species given the two species types.
11. **Shear stress estimator.** Calculates the shear stress on the particles. The input is the particles data and the flow data. The output is the value of the shear stress for each particle.
12. **pH monitoring.** The input is the reading from the pH sensor. pH variations happen due to nutrient consumption during biomass photosynthesis and depends on medium injection. The output goes directly to the chemical model.

## Claims

1. Method for growing biomass particles, in particular microalgae, in a culture medium and in the presence of one or more light and nutrient sources, **characterized in that** it predicts the space-time control of said biomass particles with respect to said light and nutrient sources, wherein said space-time control is implemented through the formation of one or more vortices in laminar flow, said vortices entraining said microalgae according to a numerically predictable trajectory, said vortices being provided through fluid dynamic simulations, in the mixture of culture medium, biomass particles and nutrients, said method providing for:
I) modelling of the geometry of the device forming said vortices;
II) calculation of the flow field of the volume of fluid forming said vortices;
III) tracking of the biomass particles;
IV) calculation of the radiative model;
V) calculation of the growth model of the biomass particles;
VI) calculation of the shear stress on the cell structure of biomass particles of different type and at different growth stages.

2. Method according to claim 1, **characterized in that** it tracks the particles through calculation models obtaining the position in space of the particles as a function of time within the flow field also in the presence of vortices.

3. Method according to claim 2, **characterized in that** it optimizes and maximizes the growth rate of the biomass of different type and at different development stages knowing the position in space of the particles as a function of time and through controlling the supply of light and nutrients to the biomass particles.

4. Method according to claim 1, **characterized in that** said modelling of the geometry of the vortex forming device includes producing a mesh, i.e., a numerical discretization, suitable to describe the geometry of the domain of the flow field of said culture medium.

5. Method according to claim 4, **characterized in that** said calculation of the flow field of the vortex formation volume of the fluid is implemented through the software OpenFOAM, using a DNS (Direct Numerical Simulation) model.

6. Method according to claim 4, **characterized in that** said calculation of the flow field of the vortex formation volume of the fluid is implemented with simplified LES (Large Eddy Simulation) models (https://en.wikipedia.org/wiki/Large_eddy_simulation) or RANS (Reynolds Averaged Navier Stokes) models (https://en.wikipedia.org/wiki/Reynolds-averaged_Navier%E2%80%93Stokes_equations), in a model for single-phase and two-phase incompressible fluids, to take into account the possible presence of air bubbles.

7. Method according to claim 1, **characterized in that** said tracking of the biomass particles is implemented with the software OpenFOAM, modifying the Lagrangian Particle Tracking model (LPT method) to take into account the characteristics of the different biomass particles dependent on species.

8. Method according to claim 1, **characterized in that** said calculation of the radiative model provides for the use of the Discrete Ordinates Method (DOM) present in the software OpenFOAM.

9. Method according to claim 1, **characterized in that** said calculation of the growth model of biomass particles is based on a system of three differential equations representing the status of the system meant as amount of biomass capable of reproducing, amount of inhibited biomass, i.e., not capable of utilizing the incident radiation, and amount of resting biomass, said model being experimentally validated by means of three multiplicative constants of the three states, so as to predict the growth of the biomass particles the parameters calculated by the aforesaid preceding models being known.

10. Method according to claim 1, **characterized in that** said calculation of the shear stress on the cell structure of biomass particles of different type and at different growth stages is implemented in the area of maximum gradient of the flow velocity of said culture medium.
